# EUROPEAN PATENT APPLICATION

(11) **EP 0 927 765 A1**
(43) Date of publication of application: **07.07.1999**
(21) Application number: 98933906.4
(22) Date of filing: 23.07.1998
(51) Int. Cl.: C12N 15/65, C12N 15/82, C12N 5/04, A01H 5/00

(54) **METHOD FOR SELECTING TRANSFORMED CELLS**

(30) Priority: 23.07.1997 JP 19662997
(71) Applicant: Japan Tobacco Inc., Tokyo 105-8422 (JP)
(72) Inventor: KOMARI, Toshihiko Japan Tobacco Inc., Shizuoka 438-080 (JP); KOMARI, Toshihiko Japan Tobacco Inc., Shizuoka 438-080 (JP); KOMARI, Toshihiko Japan Tobacco Inc., Shizuoka 438-080 (JP)
(74) Representative: Behnisch, Werner, Dr.
(86) International application number: JP9803291
(87) International publication number: WO9905296

(57) **Abstract**

In the transformation of rice, there have been employed nptII as a selective marker gene (drug resistance gene) and kanamycin, G418, etc. as selection drugs. However, only low transformation efficiency can be established thereby. It his therefore been required to establish a rice transformation system with the use of a novel combination to achieve high efficiency. According to the present invention, it is possible to transform rice with remarkably high efficiency by using paromomycin as a novel selection drug.

## Description

### FIELD OF THE INVENTION

This invention relates to a method for selecting cells which have been transformed by a desired gene. More specifically, it relates to a method for selecting transformed cells by culturing cells from rice tissue in a selective medium containing paromomycin.

### PRIOR ART

In conventional methods in order to obtain a transformed plant having a specific gene introduced therein, a selective marker gene is transferred together with the gene of interest so as to determine whether the desired gene has been transferred into the cells or not. The expression of the selective marker gene is examined to select the transformed cells into which the above-mentioned gene has been transferred. Transformed plant can be regenerated from the cells thus selected. As such marker genes, those capable of providing resistance against selection drugs, i.e., drug resistance genes, are employed. Namely, in widely employed selection methods a drug resistance gene and a gene of interest are transferred into cells simultaneously and then drug-resistant cells are selected as transformed cells. A typical example of such drug resistance genes is neomycin phosphotransferase (nptII) gene which is used in combination with kanamycin as the corresponding selection drug.

In early studies on gene transfer in rice, a combination of the above-mentioned nptII gene with kanamycin was employed (Uchimiya et al., 1986, Yang et al., 1988). In these reports, however, no regenerated transformed plant is obtained yet. Toriyama et al. (1988) reported that they could not obtain any complete green plant by way of the selection with kanamycin. Moreover, Dekeyser et al. (1989) pointed out that rice calluses were inherently resistant to kanamycin. Accordingly, there exist various problems in association with the use of the combination of nptII gene with kanamycin in selecting transformed rice cells.

On the other hand, attempts have been made to use G418 as a selection drug, which is neutralized by the expression of nptII, similarly to kanamycin. Thus, there have been several reports wherein rice transformants were obtained with the use of G418 (Toriyama et al., 1988, Peng et al., 1992, Chan et al., 1993). Toriyama et al. (1988) reported that G418 was superior to kanamycin in transformation efficiency.

Today, hygromycin phosphotransferase (hpt) gene is most widely employed as a selective marker gene in rice transformation. This gene is used in combination with hygromycin as a corresponding selection drug (Vasil 1994). It has also been reported that bar gene (selection drug: PPT and derivatives thereof) is highly effective as a selective marker gene for rice (Ayres and park 1994).

Although there have been few reports comparing selective marker genes (drug resistance genes) usable in the transformation of rice, it has been pointed out that nptII marker was less efficient than hpt marker (Shimamoto et al., 1989, Aldemita and Hodges 1996). As a matter of fact, nptII marker is not commonly used today, as described above.

Accordingly, hpt and bar genes have generally been used as selective marker genes (drug resistance genes) in the transformation of rice. In contrast, nptII gene is not often used in the transformation of rice due to poor transformation efficiency.

Although it is possible to transform rice by using hpt or bar gene, one or more different selective markers are required to be employed if one desires to transfer one or more additional genes into the transformant. That is to say, when an additional gene is to be transferred into a transformant which has been obtained by the co-transfer of a hpt or bar gene, it is necessary to use another selective marker (drug resistance) gene.

Although selection can be made by the combined use of nptII with G418, poor transformation efficiency would result. Accordingly, there has been a great demand for a novel combination of a selective marker gene (drug resistance gene) with a selection drug which provides improved transformation efficiency.

### SUMMARY OF THE INVENTION

To satisfy the above-mentioned demand, the inventors have conducted extensive studies to establish a highly efficient rice transformation system with the use of a novel combination of a drug resistance gene with a selection drug. As a result, they have found that the problem can be solved by using paromomycin as the selection drug, thus completing the present invention.

Accordingly, the present inventors have solved the above-mentioned problem by providing a method for selecting transformed cells which comprises transferring at least a structural gene of interest and a paromomycin resistance gene into cells of rice tissue, culturing the cells in a selective medium containing paromomycin, and selecting transformed cells, and the transformed cells selected by this method.

In one embodiment of the present invention, there is provided a method for selecting transformed cells which comprises the steps of:
a) providing a strain of the genus *Agrobacterium* which harbors a plasmid containing a T-DNA region which in turn contains a paromomycin resistance gene and a gene of interest in such a way as to allow the expression of said genes;
b) providing cells of rice tissue;
c) inoculating the rice cells with the above *Agrobacterium* strain;
d) culturing the inoculated cells in a medium for plant cell culture containing paromomycin at such a concentration as to allow survival of only cells that have been transformed by the paromomycin resistance gene (hereinafter referred to as a selective medium), and thus selecting paromomycin-resistant calluses; and
e) if necessary, repeating the selection step d) once or more times by using cells from the selected calluses. The cells thus selected can be cultured in a medium appropriate for regeneration of plants so that they will regenerate into complete plants.

### BRIEF DESCRIPTION OF THE DRAWINGS

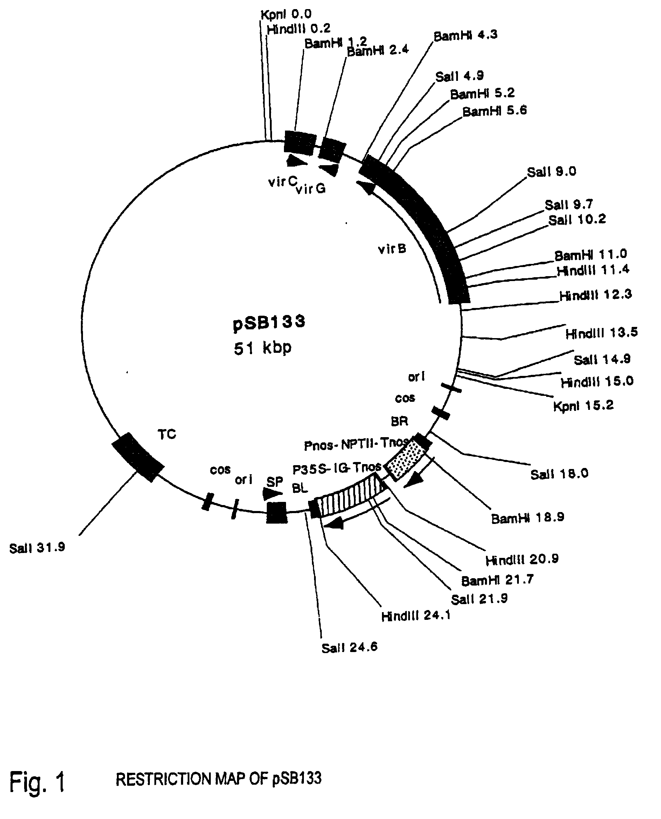

Fig. 1 shows a restriction map of the vector pSB133 employed in Example 2.

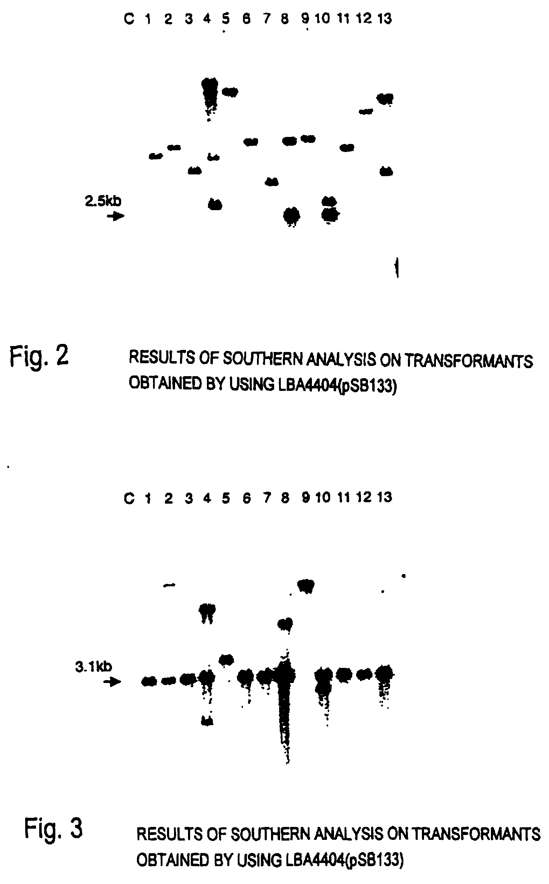

Fig. 2 shows an electrophoretic photograph showing the results of Southern analysis on the transformants obtained with the use of LBA4404(pSB133). DNA extracted from each transformant was treated with HindIII and subjected to Southern hybridization with the use of nptII gene as a probe. C: non-transformant (Asanohikari). 1-13: transformants.
Fig. 3 shows an electrophoretic photograph showing the results of Southern analysis on the transformants obtained with the use of LBA4404(pSB133). DNA extracted from each transformant was treated with HindIII and subjected to Southern hybridization with the use of the GUS gene as a probe. C: non-transformant (Asanohikari). 1-13: transformants.

### DETAILED DESCRIPTION OF THE INVENTION

Now, the present invention will be described in greater detail.

As stated above, the main features of the present invention reside in the method for selecting transformed cells which comprises transferring at least a structural gene of interest and a paromomycin resistance gene into cells of rice tissue, culturing the cells in a selective medium containing paromomycin, and selecting transformed cells, and the transformed cells selected by this method.

Paromomycin used in the invention is an aminoglucoside antibiotic which is represented by the molecular formula C₂₃H₄₅N₅O₁₄ (molecular weight; 615.5), produced by actinomycetes typified by *Sterptomyces rimosus forma parmomycinus*, and capable of inhibiting gram-positive and gram-negative bacteria. It has a structure wherein the amino group at the 6'-position of neomycin has been substituted by a hydroxyl group. It is reported that paromomycin has function and inhibition mechanisms similar to those of 2-deoxystreptamine-containing antibiotics such as kanamycin. Preparations comprising paromomycin I as the major ingredient and paromomycin II as a contaminant are commercially available as paromomycin sulfate. Such a product can be purchased from Sigma (St. Louis, USA), etc.

In accordance with the present invention, use is made of a selective medium containing paromomycin. It is preferred that the concentration of paromomycin in the selective medium ranges from 5 mg/l to 400 mg/l, still preferably from 20 mg/l to 200 mg/l and still more preferably from 40 mg/l to 100 mg/l. When the paromomycin concentration in the medium is higher than the upper limit as specified above, the growth of transformed cells will also be strongly inhibited. When the concentration is lower than the lower limit as specified above, untransformed cells will not be inhibited from growing. In both of these cases, it is therefore not be possible to select transformed cells.

Selective medium used in the present invention is not particularly limited, so long as it is selected from those conventionally used as a selective medium for rice tissue cells. Preferred examples include modified CC medium and modified MS medium (Christou et al., 1991). Still preferred are 2N6 medium and N6-7 medium, the compositions of which will be described in examples given hereinafter.

Culture in such a medium may be performed under conventional conditions.

Transformed cells to be selected by the method of the present invention may be any cells, so long as they originate from rice tissue, although cells from a rice embryo are preferred.

Transformed cells thus selected should carry at least one gene of interest, which is typified by a structural gone encoding a beneficial protein or a gene capable of imparting a favorable characteristic to rice, in addition to a paromomycin resistance gene, having been transferred thereinto.

As described above, the gene of interest is not limited to any specific one so long as it is capable of imparting a beneficial characteristic to rice. Also, the gene is not limited to one occurring in nature. That is, use may be made of a genetically engineered modified gone or a gene encoding a chimeric protein composed of plural proteins connected to each other, etc. Moreover, the gene of interest may be transferred not only in the sense direction but also in the antisense direction.

The paromomycin resistance gene is not particularly limited, so long as it is capable of neutralizing the effect of paromomycin. A gene encoding neomycin phosphotransferase (nptII) is employed herein as an appropriate example of the paromomycin resistance gene.

The two genes described above should be transferred so that they will be expressed. Accordingly, these genes are generally used together with a promoter and a terminator, etc. necessary for the expression. It is also possible to transfer two or more genes of interest or additional drug resistance gene(s), etc.

In the present invention, these genes are transferred into rice by a method commonly employed to transfer a gene into rice tissue cells. For example, any of the electroporation method, the polyethylene glycol method, the particle gun method or the *Agrobactorium-mediated* method way be employed. Among them, the *Agrobacterium*-mediated method is preferred in view of transformation efficiency. Methods for transforming cells originating from rice tissue by transferring a gene therein by the *Agrobacterium*-mediated method is described in, for example, WO 95/06722.

In the present invention, the selection of transformed cells may be conducted just one time. However, it is preferred to repeat the selection several times in order to increase the possibility of obtaining transformants. In the examples given hereinafter, the selection was repeated three times.

### Example 1

### (1) Agrobacterium strain and plasmid

As the *Agrobacterium* with the vector therein, use was made of LBA4404(pTOK233) (Hiei et al.,. 1994). The T-DNA region in the vector contained a hpt gene driven by a CaMV (cauliflower mosaic virus) 35S promoter, a nptII gene driven by a NOS (nopaline synthetase) promoter, and a GUS gene interrupted by on intron of catalase gene from castor bean.

### (2) Test variety and tissue

As a test variety, a Japanese rice variety Asanohikari was used. Tissues tested were calluses obtained from immature embryos of this variety. The immature embryos were prepared in accordance with the method of Hiei et al., (1994), and cultured on a 2N6 medium (Hiei et al., 1994) for 2 weeks. The calluses formed from the scutellum were cultured for additional 4 to 5 days on the same medium and employed as test tissues.

### (3) Inoculation and cocultivation

The inoculation and cocultivation were carried out as reported by Hiei et al. (1994). The density of the inoculum was adjusted to 1 x 10⁹ /ml.

### (4) Selection of transformed cells

After cocultivating for 3 days, calluses were transplanted onto a 2N6 medium (Hiei et al., 1994) containing either 50 mg/l of hygromycin, 400 mg/l of kanamycin, 50 mg/l of G418 or 50 mg/l of paromomycin and cultured at 30°C under light conditions for about 3 weeks. The drug-resistant calluses thus formed were transplanted onto a N6-7 media (Hiei et al., 1994) respectively containing 50 mg/l of each drug and a secondary selection was performed for 10 days. Each selective medium further contained 250 mg/l of cefotaxime. The selective media containing paromomycin and G418 contained 8 g/l of agarose as a gelling agent.

The compositions of the above-mentioned 2N-6 medium and N6-7 medium are as follows.
2N6 medium: N6 inorganic salts, N6 vitamins, 1 g/l of Casamino acid, 30 g/l of sucrose, 2 mg/l of 2,4-D (2,4-dichlorophenoxyacetic acid), 2 g/l of Gelrite, pH 5.8.
N6-7 medium: N6 inorganic salts, N6 vitamins, 2 g/l of Casamino acid, 30 g/l of sucrose, 30 9/l of sorbitol, 1 mg/l of 2,4-D, 0.5 mg/l of 6-benzyladenine, 2 g/l of Gelrite, pH 5.8.

### (5) Regeneration of transformants and examination of GUS expression

The drug-resistant embryogenic calluses obtained by the secondary selection were placed on the regeneration medium described by Abe and Futsuhara (1986), provided that the major inorganic salt concentration was reduced by half. Gelrite (4 g/l) or agarose (8 g/l) was added to the medium as a gelling agent. The regeneration media also contained one of the selection drugs each at the concentration defined above. After culturing at 25°C under light conditions for 4 to 5 weeks, leaf pieces of the drug resistant regenerated plants thus obtained were treated with X-Gluc to examine for the expression of GUS (Hiei et al., 1994). The regenerated individuals were transplanted into a 500 fold diluted aqueous solution of Hyponex and cultivated therein at 25°C under light conditions for 10 days before being transplanted into pots in a greenhouse.

### (6) Results and discussion

In the kanamycin test group, the growth of the untransformed calluses was not inhibited in spite of the high drug concentration (400 mg/l). Thus, no transformed callus could be selected (Table 1). In the G418 group, no regeneration occurred, though non-embryogenic drug resistant calluses were observed at a low frequency (Table 1).

Many of the individuals which were selected with the use of paromomycin and underwent regeneration showed a uniform GUS expression in leaves, as observed in the selection with the use of hygromycin. Thus, these individuals were confirmed to be transformants (Table 1). No albino individual was observed in any of the test groups. The transformation efficiency achieved by paromomycin was obviously higher than that in the group where the selection was conducted with hygromycin which is conventionally employed in the transformation of rice (Table 1).

These facts indicate that paromomycin is highly useful as a novel selection drug in the transformation of rice. In other words, it has been discovered that nptII gene, which was not used due to the poor efficiency, is of great use as a marker gene if it is used in combination with paromomycin in the transformation of rice.

Selection of transformants with the use of paromomycin also resulted high efficiencies in other rice varieties, i.e., Tsukinohikari and Koshihikari.

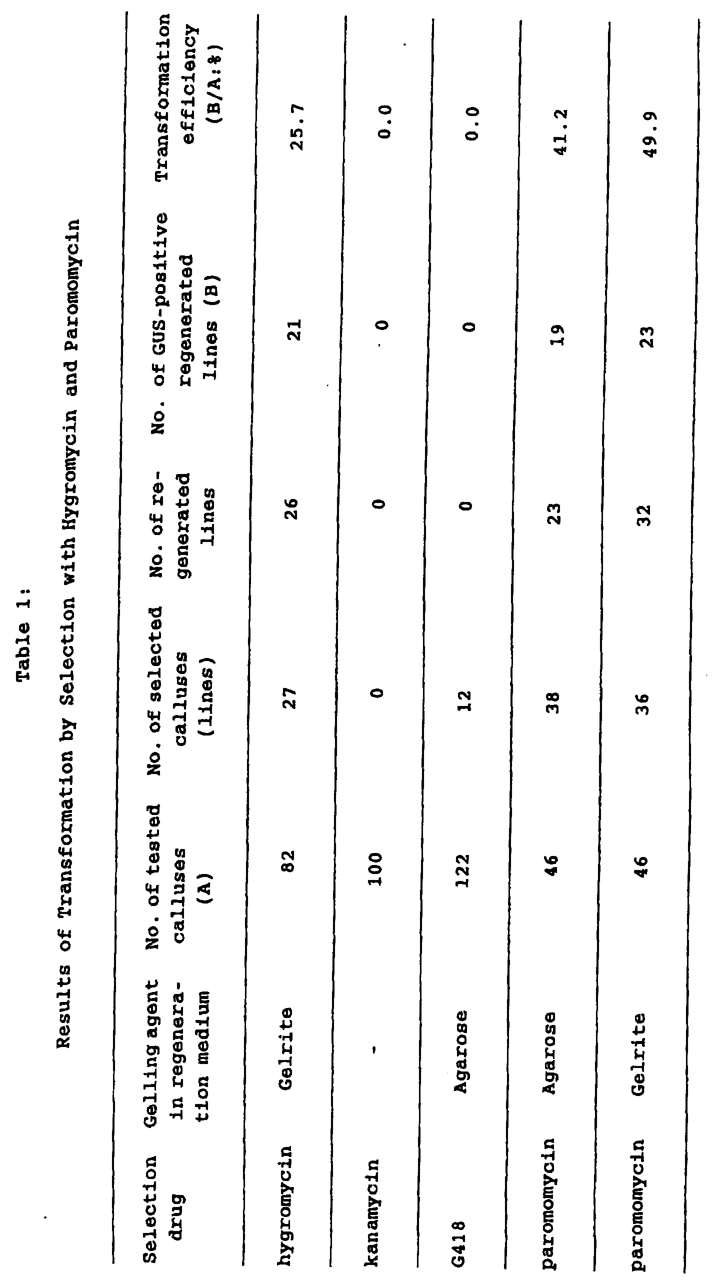

### Example 2

### (1) Vector

LBA4404(pSB133) (Fig. 1) was used as *Agrobacterium* strain containing the plasmid vector to transform Japonica rice varieties Asanohikari and Tsukinohikari. In the T-DNA region, this vector had a npt gene under control of a NOS promoter, and a GUS gene interrupted by an intron of catalase gene from castor bean. The transformation was carried out by the method described above. The regeneration medium contained 4 g/l of Gelrite as a gelling agent.

pSB133 was constructed in the following manner. A DNA fragment (6.2 kb) obtained by digesting pGA482 (An et al., 1985) with SaII was ligated to another DNA fragment (5.1 kb) obtained by digesting pSB11 (Komari et al., 1996) with SalI to give a plasmid. Next, this plasmid was digested with EcoRI and BglII to give a DNA fragment (8.6 kb). This DNA fragment was blunt-ended and a BglII linker (manufactured by Takara) was inserted thereinto go give plasmid pSB27. The plasmid pSB27 was digested with HindIII to be subsequently ligated with a fragment (3.1 kb) containing a 355 promoter and an intron-interrupted GUS gene, which had been obtained by digesting pIG221 (Ohta et al., 1990) with HindIII. Thus, pSB33 was obtained. The plasmid pSB33 was introduced into *Escherichia. coli* strain LE392, and then transferred into *Agrobacterium* strain LBA containing pSB1 (komari et al., 1996) by the triparental mating method (Ditta et al., 1980). The plasmid pSB133 was formed via the homologous recombination between pSB1 and pSB33 in the *Agrobacterium*.

### (2) Transformation

The transformation with LBA4404(pSB133) yielded drug resistant calluses at a high frequency on the paromomycin-containing medium, similar to the case with the use of LBA4404(pTOK233). The calluses could regenerate on the paromomycin-containing medium without difficulty. Many of the thus regenerated individuals showed a uniform GUS expression in leaves, which indicated that they were transformants (Table 2).

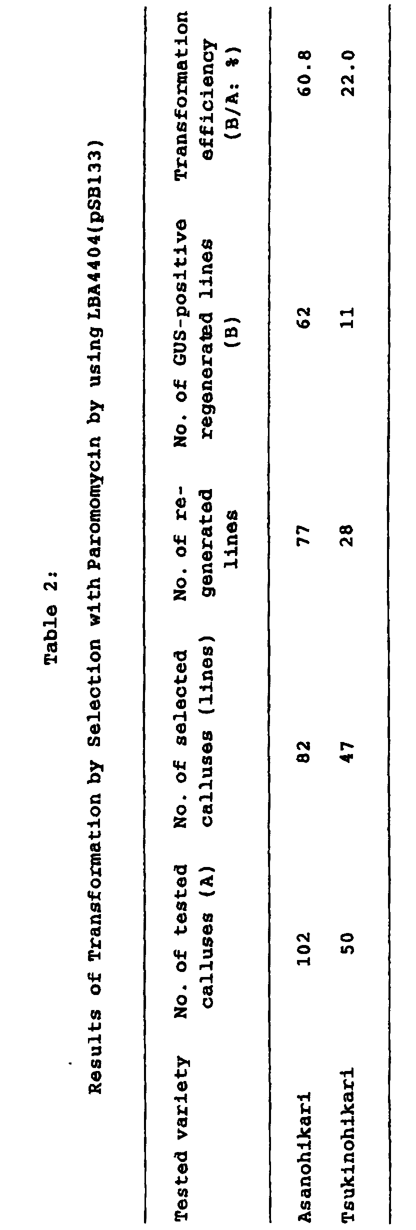

### (3) Confirmation of gene transfer

DNA was extracted from the leaves of 13 paromomycin-resistant and GUS-positive individuals derived from Asanohikari by the inoculation with LBA4404 (pSB133). The extracted DNA was treated with HindIII and subjected to Southern hybridization with the use of nptII gene or GUS gene as a probe. As a control, DNA from untransformed Asanohikari was used. Southern hybridization was performed as described in Molecular Cloning (Sambrook et al., 1909).

The Southern analysis of the HindIII-digested DNA with the use of nptII gene as a probe showed one to several copies of the transferred gene in all of the 13 individuals tested (Fig. 2). In the plasmid pSB133, the HindIII fragment containing nptII gene showed constantly the size of 5.9 kb (Fig. 1). Therefore, if the *Agrobacterium* had remained in the rice plants, only the band of this size should have been detected. The fact was, bands differing in size were detected from the tested transformants (Fig. 2). Further, most of these bands were longer (Fig. 2) than the region extending from the HindIII site, through the nptII gene, to the left border (about 2.5 kb, Fig. 1). These results indicated that T-DNA was integrated into different chromosomal regions in the individuals tested, suggesting that these individuals were independent transformants.

The Southern analysis of the HindIII-digested DNA with the use of GUS gene as a probe showed a band of 3.1 kb in 11 out of 13 transformants (Fig. 3). In the plasmid pSB133, the HindIII fragment containing the GUS gene showed constantly the size of 3.1 kb (Fig. 1). Thus, the results of the Southern analysis agreed with the predicted band length. Bands with different lengths were obtained from some individuals, which was seemingly caused by the rearrangement of the transferred gene. This phenomenon is frequently observed also in dicotyledonous transformants obtained by the *Agrobacterium*-mediated method (Deroles and Gardner, 1988; Komari, 1989; Komari, 1990).

When the expression of paromomycin resistance and GUS gene was examined in self-fertilized individuals of the next generation of the transformants, genetic segregation based on Mendel's law was confirmed.

Previously, in the transformation of rice with the use of nptII as a selective marker gene, kanamycin and G418 were employed as selection drugs. However, only low transformation efficiencies could be achieved by these drugs. According to the present invention, it is possible to transform rice tissue cells with remarkably high efficiency by using paromomycin as a novel selection drug.

### References

1) Abe, T. and Futsuhara, Y. (1986) Plant regeneration from suspension culture of rice. Jap. J. Breed. 36:1-6.
2) Aldemita, R. R. and Hodges, T. K. (1996) *Agrobacterium* tumefaciens-mediated transformation of japonica and indica rice varieties. Planta 199: 612-617
3) Ayres, N. M. and Park, W. D. (1994) Genetic transformation of rice. Critical Reviews in Plant Science 13(3): 219-239.
4) Chan, M. T., Chang, H. H., Ho, S. L., Tong, W.F. and Yu, S. M. (1993) *Agrobacterium*-mediated production of transgenic rice plants expressiong a chimeric alpha-amylasepromoter/beta-glucuronidase gene. Plant Mol. Biol. 22:491-556.
5) Christou, P., Ford, T. L. and Kofron, M. (1991) Production of transgenic rice (*Oryza sativa L.*) plants from agronomically important indica and japonica varieties via electric discharge particle acceleration of exogenous DNA into immature zygotic embryos. Bio/technology 9:957-962.
6) Dekeyser, R., Claes, B., Marichal, M., Van Montagu, M. and Caplan, A. (1989) Evaluation of selectable markers for rice transformation. Plant Physiol. 90:217-223.
7) Hiei Y., Ohta, S., Komari, T. and Kumashiro, T. (1994) Efficient transformation of rice (*Oryza Sativa L.*) mediated by transformation by *Agrobacterium* and sequence analysis of the boundaries of the T-DNA. The Plant Journal 6:271-282.
8) Peng, J., Kononowicz, H. and Hodges, T. K.(1992) Transgenic indica rice plants. Theor. Appl. Genet. 83:855-863.
9) Shimamoto, K.. Terada, R., Izawa, T. and Fujimoto, H., (1989) Fertile transgenic rice plants regenerated from transformed protoplasts. Nature 338:274-276.
10) Toriyama, K., Arimoto, Y., Uchimiya, H. and Hinata, K., (1988) Transgenic rice plants after direct gene transfer into protoplasts. Bio/Technology, 6:1072-1074.
11) Uchimiya, H., Fushimi, T., Hayashimoto, H., Harada, H., Syono, K. and Sugawara, Y., (1986) Expression of a foreign gene in callus derived from DNA-treated protoplasts of rice (*Oryza sativa L.*). Mol. Gen. Genet. 204:204-.
12) Vasil, I. K. (1994) Molecular improvement of cereals. Plant Mol. Biol. 25: 925-937.
13) Yang. H., Zhang H.M., Davey, M. R., Mulligan. B. J., and Cocking, E. C., (1988) Production of kanamycin resistant rice tissues following DNA uptake into protoplasts. Plant Cell Rep. 7:421-.
14) An, G., Watson, B. D., Stachel, S., Gordon, M. P., and Nester, E. W. (1985). New cloning vehicles for transformation of higher plants. EMBO J 4, 277-288.
15) Deroles, S. C., and Gardner, R. C. (1988). Analysis of the T-DNA structure in a large number of transgenic petunias generated by *Agrobacterium*-mediated transformation. Plant. Mol. Biol. 11, 365-377.
16) Ditta, G., Stanfield, S., Corbin, D., and Helinski, D. R. (1980). Broad host range DNA cloning system for Gram-negative bacteria: Construction of gene bank of *Rhizobium meliloti*. Proc. Natl. Acad. Sci. USA 77, 7347-7351.
17) Komari, T. (1989). Transformation of callus cultures of nine plant species mediated by Agrobacterium. Plant Sci. 60, 223-229.
18) Komari, T. (1990). Genetic characterization of double-flowered tobacco plant obtained in a transformation experiment. Theor. Appl. Genet. 80, 167-171.
19) Komari, T., Hiei, Y., Saito, Y., Murai, N., and Kumashiro, T. (1996). Vectors carrying two separate T-DNAs for co-transformation of higher plants mediated by *Agrobacterium tumefaciens* and segregation of transformants free from selective markers. Plant J 10, 165-174.
20) Ohta, S., Mita, S., Hattori, T., and Nakamura, K. (1990). Construction and expression in tobacco of a β-glucuronidase (GUS) reporter gene containing an intron within the coding sequence. Plant Cell Physiol. 31, 805-813.
21) Sambrook, J., Fritsch, E. F., and Maniatis, T. (1989). Molecular Cloning: A Laboratory Manual, 2nd Edition (Cold Spring Harbor, NY: Cold Spring Harbor Laboratory Press).

## Claims

1. A method for selecting transformed cells which comprises culturing cells originating from rice tissue in a selective medium containing paromomycin, after transferring at least a desired structural gone and a paromomycin resistance gene into the cells, and selecting transformed cells.

2. The method for selecting transformed cells as claimed in claim 1, wherein said paromomycin resistance gene is a gene encoding neomycin phosphotransferase (nptII).

3. A method for selecting transformed cells as claimed in claim 1 or 2, wherein said transformation is carried out by the *Agrobacterium*-mediated method.

4. A method for selecting transformed cells as claimed in any of claims 1 to 3, wherein maid transformed cells are cells originating from rice embryo.

5. A method for selecting transformed cells as claimed in any of claims 1 to 4, wherein the paromomycin concentration in said selective medium ranges from 5 mg/l to 400 mg/l.

6. A method for selecting transformed cells as claimed in any of claims 1 to 5, wherein said selective medium is 2N6 medium or N6-7 medium.

7. A transformed rice cell which is selected from cells originating from rice tissue by culturing the cells in a selective medium containing paromomycin after at least a desired structural gene and a paromomycin resistance gene were transferred into the cells.

8. A method for producing a rice transformant transformed by a desired gene comprising:
a) providing a strain belonging to the genus *Agrobacterium* which has a plasmid containing a paromomycin resistance gene and a desired gone, in the T-DNA region in said plasmid, in such a way as to allow expression of each of said genes;
b) providing cells originating from rice tissue;
c) inoculating the above cells originating from rice with the above strain belonging to the genus *Agrobacterium*;
d) culturing the inoculated cells in a culture medium for plant cells containing paromomycin at such a concentration that will not allow cells other than those having been transformed by the paromomycin resistance gene to survive in said medium (hereinafter referred to as a selective medium), and selecting paromomycin-resistant calluses;
e) if necessary, repeating the selection step d) once or more by using tissue cells obtained from the selected calluses; and
f) culturing the selected calluses in a medium appropriate for plant regeneration to give a completely regenerated plant.

9. The method as claimed in claim 8, wherein said cells in the step b) are those originating in rice embryo.

10. The method as claimed in claim 8 or 9, wherein said paromomycin resistance gene is a gene encoding neomycin phosphotransferase (nptII).

11. The method as claimed in claim 10, wherein the paromomycin concentration in said selective medium ranges from 5 mg/l to 400 mg/l.

12. The method an claimed in claim 11, wherein said selective medium is 2N6 medium or N6-7 medium.

13. The method as claimed in claim 8, wherein said inoculation in the step c) is performed by culturing said cello originating in rice in the presence of said strain belonging to the genus *Agrobacterium* for several minutes to several days.

14. The method as claimed in claim 8, wherein one to several copies of said desired gene are integrated into the genome of the plant thus regenerated.
